Europäisches Patentamt

European Patent Office

Office européen des brevets

(11) Veröffentlichungsnummer: **0 041 621**
**B1**

(12) **EUROPÄISCHE PATENTSCHRIFT**

(45) Veröffentlichungstag der Patentschrift:
27.03.85

(51) Int. Cl.⁴: **C 01 B 33/28**, B 01 J 29/04,
B 01 J 29/28

(21) Anmeldenummer: 81103575.7

(22) Anmeldetag: 11.05.81

(54) Verfahren zur Herstellung von Zeolithen.

(30) Priorität: 07.06.80 DE 3021580

(43) Veröffentlichungstag der Anmeldung:
16.12.81 Patentblatt 81/50

(45) Bekanntmachung des Hinweises auf die Patenterteilung:
27.03.85 Patentblatt 85/13

(84) Benannte Vertragsstaaten:
BE DE FR GB IT NL

(56) Entgegenhaltungen:
EP - A - 0 007 081
EP - A - 0 009 976
DE - A - 2 442 240
DE - A - 2 817 575
DE - A - 2 817 576
US - A - 3 702 886

(73) Patentinhaber: **BASF Aktiengesellschaft,
Carl-Bosch-Strasse 38, D-6700 Ludwigshafen (DE)**

(72) Erfinder: **Marosi, Laszlo, Dr., Leuschnerstrasse 32,
D-6700 Ludwigshafen (DE)**
Erfinder: **Schilmper, Hans-Ulrich, Dr., Im Erlich 74,
D-6720 Speyer (DE)**
Erfinder: **Schwarzmann, Matthias, Dr.,
Carl-Bosch-Strasse 54, D-6703 Limburgerhof (DE)**
Erfinder: **Stabenow, Joachim, Dr., Am Feldrain 22,
D-6940 Weinheim (DE)**

BUNDESDRUCKEREI BERLIN

0 041 621

## Beschreibung

Die Erfindung betrifft ein Verfahren zur Herstellung von Zeolithen vom Strukturtyp ZSM-5. Zeolithe des A-, X-, Y- und Mordenit-Typs haben eine große technische Bedeutung erlangt. Sie werden als Ionenaustauscher, Molekularsiebe und Katalysatoren technisch eingesetzt. Technische Verfahren, wie das katalytische und hydrierende Cracken von Kohlenwasserstoffen werden mit Zeolithkatalysatoren durchgeführt. Neuerdings gewinnen Zeolithe vom Typ ZSM-5 zunehmend an Interesse, z. B. für Reaktionen wie die Umwandlung von Methanol in Aromaten und/oder Olefine.

Zeolithe sind kristalline Silikate, insbesondere Aluminosilikate. Zur Herstellung von Aluminosilikatzeolithen werden reaktive $SiO_2$- und $Al_2O_3$-Ausgangsmaterialien in Gegenwart von starken Basen hydrothermal zur Kristallisation gebracht. Als starke Basen verwendet man bei den bekannten Verfahren in der Regel Alkali- oder Erdalkalihydroxide oder quaternäre Stickstoff- oder Phosphorbasen, entweder allein oder in Mischungen miteinander.

Zeolithe vom Typ ZSM-5 wurden zuerst aus Mischungen von reaktivem $SiO_2$ und $Al_2O_3$ durch hydrothermale Kristallisation in Gegenwart von Tetrapropylammonium- und Natriumionen hergestellt (US-A-3 702 886). Ein Nachteil dieser Synthesemethode besteht in der Verwendung von schwer zugänglichen quaternären Stickstoffbasen und darin, daß die Alkalikationen gegen andere Kationen ausgetauscht werden müssen, damit die katalytisch aktive Form entsteht. Es ist bekannt, daß die Alkaliionen nur durch einen besonders aufwendigen Ionenaustausch aus den Hohlräumen von ZSM-5 zu entfernen sind (DE-A-2 442 240).

Die weitere Entwicklung der Synthese von Zeolithen vom ZSM-5-Typ war daher auf die Beseitigung der obengenannten Nachteile gerichtet. Man hat auch schon primäre Amine oder Diamine in Verbindung mit Alkali an Stelle der quaternären Stickstoffbasen bei der Herstellung von ZSM-5-artigen Zeolithen verwendet. Diese bekannten Verfahren sind in DE-A-2 442 240, 2 817 575 und 2 817 576 beschrieben. Ein gemeinsames Merkmal dieser Herstellungsverfahren besteht darin, daß auch diese Synthese in Gegenwart von Alkaliionen erfolgt.

Aus EP 7081 ist auch ein Verfahren bekannt, Zeolithe mit Hilfe von Hexamethylendiamin aus Mischungen von $SiO_2$ und $Al(OH)_3$ in Abwesenheit von Alkali herzustellen. Bei der Verwendung von anderen Aminen anstelle von Hexamethylendiamin hat sich jedoch gezeigt, daß nicht alle beliebigen Mengenverhältnisse in Synthesemischungen zu kristallinen Produkten führen bzw. nur schlecht kristallisierte Produkte ergeben.

Der vorliegenden Erfindung liegt die Aufgabe zugrunde, aus billigen Ausgangsmaterialien neue, katalytisch aktive Zeolithe zu synthetisieren, die in Na-freier Form entstehen und daher ohne vorangehenden Ionenaustausch, allenfalls nach einer einfachen Kalzinierung, direkt als Katalysatoren eingesetzt werden können.

Die vorliegende Erfindung betrifft daher ein Verfahren zur Herstellung von Zeolithen vom Strukturtyp ZSM-5, durch hydrothermale Kristallisation bei Temperaturen von 100 bis 200°C aus Mischungen von $SiO_2$ und $Al(OH)$ in Abwesenheit von Alkali in wäßrigen Lösungen von Aminen, indem man die Kristallisation in Gegenwart von Triaminen, Tetraaminen und/oder höheren Aminen durchführt und dabei in der Reaktionsmischung in Abhängigkeit von der Kettenlänge $L_R$ und Zahl der Amingruppen $n$ des verwendeten Amins ein $SiO_2/Al_2O_3$-Molverhältnis zwischen

$$\frac{96 - \dfrac{20\,n}{L_R + 4}}{\dfrac{20\,n}{L_R + 4}} \quad \text{und} \quad \frac{96 - \dfrac{20\,n}{L_R + 4}}{\dfrac{4\,n}{L_R + 4}}$$

und die molaren Verhältnisse von $H_2O$/Amin im Bereich von 0,4 bis 100 und Amin/$Al_2O_3$ im Bereich von 5 bis 400 einstellt. Ausgenommen sind $SiO_2$-Molverhältnisse von 38 in Gegenwart von Dihexamethylentriamin und 26 in Gegenwart von Dipropylentriamin.

Das erfindungsgemäße Verfahren wird vorteilhaft in Gegenwart von organischen Aminen, die eine wesentlich schwächere Basizität als Alkali oder quaternäre Stickstoffbasen aufweisen, z. B. Dipropylentriamin, Dihexamethylentriamin, Diethylentriamin, Triethylentetramin oder in Gegenwart von Mischungen dieser Amine durchgeführt. Die $SiO_2/Al_2O_3$-Verhältnisse variieren dabei in charakteristischer Weise gemäß der obengenannten Formel.

Ausgenommen vom Schutz in dem vorliegenden Patent sind Verfahren, die mit $SiO_2/Al_2O_3$-Werten am unteren Grenzbereich

$$\frac{96 - \dfrac{20\,n}{L_R + 4}}{\dfrac{20\,n}{L_R + 4}}$$

2

**0 041 621**

liegen und in der älteren EP-A-34 727 bereits geschützt sind.

Durch Entfernen der Aminkomponente kann man in einfacher Weise die H-Form der Zeolithe gewinnen. Die erfindungsgemäß hergestellten Zeolithe, insbesondere deren H-Form werden als Katalysatoren für die Umwandlung von Alkoholen und/oder Dialkyläthern in Olefine und/oder Aromaten verwendet.

Bei dem erfindungsgemäßen Verfahren kann man Mischungen von reaktivem $SiO_2$, wie pyrogene Kieselsäure (Aerosil) und aktivem Aluminiumoxid, beispielsweise frisch gefälltem $Al(OH)_3$, in Gegenwart von organischen Triaminen, Tetraminen oder höher kondensierten Aminen unter hydrothermalen Bedingungen bei Temperaturen von 100 bis 200°C, insbesondere 140 bis 170°C kristallisieren.Die Konzentration der wäßrigen Aminlösungen sollte zweckmäßig im Bereich von 10 bis 70, insbesondere zwischen 40 bis 60% betragen.

Man kann das Aluminium auch teilweise durch andere dreiwertige Elemente, insbesondere Bor und/oder Eisen, ersetzen. Der Einbau von Bor in das Kristallgerüst wird durch Verschiebung der d-Werte der Röntgenbeugungslinien nach kleineren Werten angezeigt.

Eine vorteilhafte Ausführungsform des erfindungsgemäßen Verfahrens besteht darin, daß man eine Reaktionsmischung aus $SiO_2$ und Aluminiumoxid bzw. -hydroxid oder deren natriumfreie Vorläufer in dem oben angegebenen Verhältnis mischt und dann in einer wäßrigen Aminlösung 2 Stunden bis 8 Tage auf Temperaturen zwischen 100 und 200°C, vorzugsweise 1 bis 4 Tage auf Temperaturen von 140 bis 170°C unter ihrem Eigendruck erhitzt. Die so hergestellten Zeolithe enthalten in der Regel größere Mengen des verwendeten Amins in den intrakristallinen Poren eingelagert. Dieses Amin kann beispielsweise durch Verbrennen aus den Poren entfernt werden, wobei die katalytisch aktive Wasserstofform gebildet wird.

Es ist zweckmäßig, die Mutterlauge ganz oder teilweise wieder zur Herstellung von neuem Zeolith einzusetzen.

Die nachfolgende Tabelle 1 gibt den gesetzmäßigen Zusammenhang zwischen der Kettenlänge des verwendeten Amins und dem maximalen Gehalt an Aluminium in der Kristallisatmischung als Ergebnis der Kristallisationsversuche wieder.

3

| | $L_R$ (Å) | $\dfrac{96-\dfrac{20\,n}{L_R+4}}{\dfrac{20\,n}{L_R+4}}$ | $\dfrac{96-\dfrac{20\,n}{L_R+4}}{\dfrac{4\,n}{L_R+4}}$ | Kristallisations-versuche mit SiO$_2$/Al$_2$O$_3$-Molverhältnis | Produkt |
|---|---|---|---|---|---|
| Triethylentetramin | 13,4 | 19,9 | 99,5 | 15 | amorph |
| | | | | 23 | ZSM-5 |
| | | | | 90 | ZSM-5 |
| | | | | 120 | ZBM-30 |
| Diethylentriamin | 9,4 | 20,3 | 101,5 | 23 | ZSM-5 |
| | | | | 100 | ZSM-5 und |
| | | | | | ZBM-30 |
| Dihexamethylentriamin | 20 | 37,4 | 187 | 40 | ZSM-5 |
| | | | | 160 | ZSM-5 |
| Dipropylentriamin | 12 | 24,6 | 123 | 28 | ZSM-5 |
| | | | | 60 | ZSM-5 |
| Propylendiamin | 6,7 | 24,7 | 123,5 | 15 | Ferrierit |
| | | | | 25 | ZSM-5 |
| | | | | 70 | ZSM-5 |
| | | | | 100 | ZBM-30 und |
| | | | | | ZSM-5 |
| | | | | 150 | ZBM-30 |

## Beispiel 1

In 1200 g 50%ige Dipropylentriaminlösung werden 101 g Aerosil und frisch gefälltes Al(OH)$_3$ einge-tragen. Das Al(OH)$_3$ wird aus 49 g Al(NO$_3$)$_3$ · 9 H$_2$O durch Fällen mit Ammoniak hergestellt. Die Mischung wird dann so lange gerührt, bis sie homogen ist und anschließend 7 Tage in einem Stahlautoklaven auf 170°C erhitzt. Das kristalline Produkt wird filtriert, gewaschen und bei 100°C getrocknet. Laut Röntgenanalyse besteht es aus gut kristallisiertem Aluminiumzeolith vom Pentasil-Typ.

## Beispiel 2

Es werden Mischungen von SiO$_2$ und Al(OH)$_3$ hergestellt und in der im Beispiel 1 beschriebenen Weise 101 g SiO$_2$ und dem jeweiligen SiO$_2$/Al$_2$O$_3$-Molverhältnis entsprechende Menge Al(OH)$_3$ in 1200 g 50%iger Aminlösung 7 Tage bei 170°C hydrothermal behandelt. Die Zusammensetzung der Reaktionsmischung, Kristallisationsbedingungen und das Ergebnis der Versuche sind in der Tabelle 2 zusammengestellt

## 0 041 621

| R | Molverhältnis $SiO_2/Al_2O_3$ | Produkt |
|---|---|---|
| Dipropylentriamin | 20 | amorph |
| | 26 | ZSM-5 |
| | 60 | ZSM-5 |
| Dihexamethylentriamin | 40 | ZSM-5 |
| | 160 | ZSM-5 |
| | 6000 | ZBM-30 |
| Triethylentetramin | 22 | ZSM-5 |
| | 90 | ZSM-5 |
| | reines $SiO_2$ | ZBM-30 |
| Diethylentriamin | 23 | ZSM-5 |
| | 100 | ZSM-5 und ZBM-30 |

### Beispiel 3

100 g des in Beispiel 2 mit Hilfe von Triethylentetramin erhaltenen Zeoliths, $Si/Al_2 = 22$, werden mit Böhmit zu 1-mm-Strängen verarbeitet. Der Zeolithgehalt der Stränge beträgt 65 Gew.-%. 20 g des erhaltenen Produkts werden als Katalysator in einem Durchflußreaktor eingebaut und die Aktivität in der Umwandlung von Methanol in Olefine getestet.

### Herstellung von Olefinen aus Methanol

Methanol mit einem Wassergehalt von 75 Gew.-% wird bei 330° C über den Katalysator geleitet. Die Belastung beträgt 360 g Lösung/h und 20 g Katalysator. Die Reaktionsbedingungen sind in der folgenden Tabelle zusammengefaßt:

| | |
|---|---|
| Eingangstemperatur | 330° C |
| Druck | 1,16 bar |
| Umsetzung | 100% |

Das erhaltene Reaktionsprodukt hat folgende Zusammensetzung: 10 Gew.-% flüssige Kohlenwasserstoffe und 90 Gew.-% gasförmige Reaktionsprodukte, bezogen auf den $CH_2$-Wert des eingesetzten Methanols. Die gasförmigen Produkte enthalten 26 Gew.-% $C_2H_4$ und 28 Gew.-% $C_3H_6$.

5

**Patentansprüche**

1. Verfahren zur Herstellung von Zeolithen vom Strukturtyp ZSM-5 durch hydrothermale Kristallisation bei Temperaturen von 100 bis 200°C aus Mischungen von $SiO_2$ und $Al(OH)_3$ in Abwesenheit von Alkali in wäßrigen Lösungen von Aminen, dadurch gekennzeichnet, daß man die Kristallisation in Gegenwart von Triaminen, Tetraminen und/oder höheren Aminen durchführt und dabei in der Reaktionsmischung in Abhängigkeit von der Kettenlänge $L_R$ und Zahl der Amingruppen n des verwendeten Amins ein $SiO_2/Al_2O_3$-Molverhältnis zwischen

$$\frac{96 - \dfrac{20\,n}{L_R+4}}{\dfrac{20\,n}{L_R+4}} \quad \text{und} \quad \frac{96 - \dfrac{20\,n}{L_R+4}}{\dfrac{4\,n}{L_R+4}}$$

und die molaren Verhältnisse von $H_2O$/Amin im Bereich von 0,4 bis 100 und Amin/$Al_2O_3$ im Bereich von 5 bis 400 einstellt, ausgenommen $SiO_2/Al_2O_3$-Molverhältnisse von 38 in Gegenwart von Dihexamethylentriamin und 26 in Gegenwart von Dipropylentriamin als Lösungsmittel.

2. Verfahren zur Herstellung von Zeolithen nach Anspruch 1, dadurch gekennzeichnet, daß man durch Entfernen der Aminkomponente die H-Form der Zeolithe gewinnt.

**Claims**

1. A process for the preparation of zeolites of the ZSM-5 structural type from a mixture of $SiO_2$ and $Al(OH)_3$ by hydrothermal crystallization in an aqueous amine solution, in the absence of alkali, at from 100 to 200°C, wherein the crystallization is carried out in the presence of a triamine, tetraamine and/or higher amine and the rection mixture is formulated to have an $SiO_2/Al_2O_3$ molar ratio which depends on the chain length $L_R$ and number of amine groups n of the amine employed and is from

$$\frac{96 - \dfrac{20\,n}{L_R+4}}{\dfrac{20\,n}{L_R+4}} \quad \text{and} \quad \frac{96 - \dfrac{20\,n}{L_R+4}}{\dfrac{4\,n}{L_R+4}}$$

as well as to have an $H_2O$/amine molar ratio of from 0.4 to 100 and an amine/$Al_2O_3$ molar ratio of from 5 to 400, excluding an $SiO_2/Al_2O_3$ molar ratio of 38 in the presence of dihexamethylenetriamine and of 26 in the presence of dipropylenetriamine as solvent.

2. A process for the preparation of a zeolite, as claimed in claim 1, wherein the H-form of the zeolite is obtained by removing the amine component.

**Revendications**

1. procédé pour la préparation de zéolites de type structural ZSM-5 par cristallisation hydrothermique à des températures de 100 à 200"C à partir de mélanges de $SiO_2$ et d'$Al(OH)_3$, en l'absence d'alcalis et dans des solutions aqueuses d'amines, caractérisé en ce qu'on effectue la cristallisation en présence de triamines, de tétramines et/ou d'amines supérieures, en réglant dans le mélange réactionnel, en fonction de la longueur de chaîne $L_R$ et du nombre des groupes amine ne de l'amine utilisée, un rapport molaire $SiO_2/Al_2O_3$ compris entre

$$\frac{96 - \dfrac{20\,n}{L_R+4}}{\dfrac{20\,n}{L_R+4}} \quad \text{et} \quad \frac{96 - \dfrac{20\,n}{L_R+4}}{\dfrac{4\,n}{L_R+4}}$$

et les rapports molaires $H_2O$/amine dans la gamme de 0,4 à 100 et amine/$Al_2O_3$ dans la gamme de 5 à 400, à l'exclusion des rapports molaires $SiO_2/Al_2O_3$ de 38 en présence de dihexaméthylène-triamine comme solvant et de 26 en présence de dipropylène-triamine.

2. Procédé pour la préparation de zéolites selon la revendication 1, caractérisé en ce qu'on obtient la forme H des zéolites par élimination des constituants amine.